# EUROPEAN PATENT SPECIFICATION

(11) **EP 4 404 988 B1**
(45) Date of publication and mention of the grant of the patent: **29.10.2025**
(21) Application number: 22790271.5
(22) Date of filing: 16.09.2022
(51) Int. Cl.: A61L 31/04, A61L 31/14

(54) **NON-ADHESIVE COLLAGEN-LIKE HYDROGELS**
NICHTKLEBENDE COLLAGENARTIGE HYDROGELE
HYDROGELS NON ADHÉSIFS DE TYPE COLLAGÈNE

(30) Priority: 20.09.2021 EP 21197704
(43) Date of publication of application: 31.07.2024
(73) Proprietor: Evonik Operations GmbH, 45128 Essen (DE)
(72) Inventor: WEBER, Sven, 69221 Dossenheim (DE); MONTERO MIRABET, Maria, 64367 Mühltal (DE)
(74) Representative: Evonik Patent Association
(86) International application number: PCT/EP2022/075736
(87) International publication number: WO 2023/041689

(56) References cited:
- EP-B1- 2 976 352
- WO-A1-2019/046943
- AU-A1- 2014 317 807
- US-A1- 2006 258 560

## Description

### Field of the invention

The present invention pertains to a hydrogel, obtained by reacting at least one collagen-like protein and at least one cross-linker comprising at least two groups each comprising a polyalkylene glycol moiety and a succinimidyl group. Furthermore, the present invention refers to the use of the hydrogel according to the present invention as stent, for wound sealing, drug delivery, healing promotion, cell culture, spheroid production, embryoid body formation, anti-adhesion barriers, coatings, sponges, and antibacterial dressings.

### Background

Collagen is the most abundant protein in the human body. It has multiple functions including scaffold production and cell binding. It has good biocompatibility and can be widely used for medical applications and cosmetics. Collagen hydrogels are commonly used in medical applications for example as stent, for wound sealing, drug delivery, healing promotion, cell culture, spheroid production, embryoid body formation, anti-adhesion barriers, coatings, sponges, and antibacterial dressings. However, conventional collagen hydrogels can have immunogenic properties and can transmit diseases from the animal from which it is derived. Furthermore, animalderived hydrogels commonly contain many cell binding sites, which play a crucial role during natural wound healing process. The wound healing process includes a cell activation as well as a blood clotting process leading to fibrin formation finally building healed tissue layers. This natural process can lead not only to undesired tissue adhesion between muscles and organs during surgery, but as well to biofouling issues or even thrombosis.

The inventors of the present invention have surprisingly found that hydrogels, obtained by reacting at least one collagen-like protein and at least one cross-linker comprising at least two groups each comprising a polyalkylene glycol moiety and a succinimidyl group can overcome one or more of the above-mentioned disadvantages of conventional collagen hydrogels. Furthermore, the starting material for the hydrogel synthesis (the collagen-like protein) according to the present invention is less immunogenic, vegan, thus doesn't transmit diseases from animals, can be dissolved in neutral pH values at in higher concentrations with a lesser viscosity compared to conventional collagen types with a similar final concentration, can be sterile filtrated and yields in non-adhesive hydrogels after gelation. WO 2019/046943 A1 discloses said collagen-like proteins and their medical uses.

Surprisingly, the hydrogels of the current invention show an anti-fouling effect.

### Summary of the invention

Therefore, in a first aspect, the present invention refers to a hydrogel obtained by reacting at least one collagen-like protein and at least one cross-linker comprising at least two groups each comprising a polyalkylene glycol moiety and a succinimidyl group, wherein the at least one bacterial collagen-like protein is a polypeptide that is at least ≥ 60%, identical to the amino acid sequence of SEQ ID NO:1, SEQ ID NO:2, SEQ ID NO:3, SEQ ID NO:4, SEQ ID NO:5, SEQ ID NO:6, SEQ ID NO:7, SEQ ID NO:8 or SEQ ID NO:9.

In a second aspect, the present invention pertains to the use of the hydrogel according to the present invention as stent, as hydrogel for wound sealing, drug delivery hydrogel, hydrogel for healing promotion, anti-adhesion barrier, coating, sponge, antibacterial dressing, or in cell culture, spheroid production and embryoid body formation.

### Description of the figures

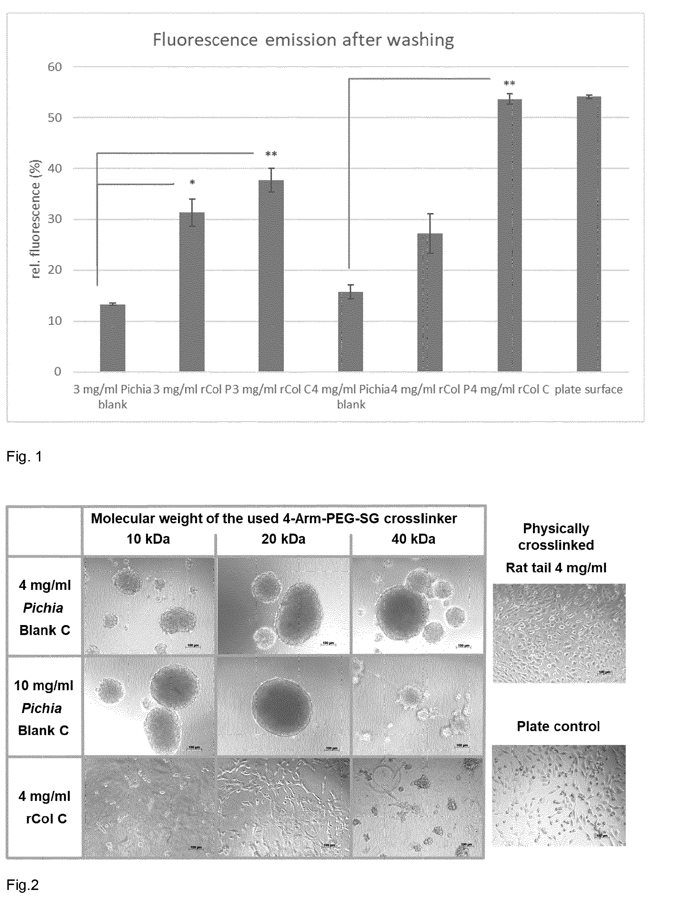

**Fig. 1****:** Quantified fluorescence emission of attached cells of figure 1 (n = 3). The lower the signal, the less cells attached. Significance was calculated using a Student's T-test (2 dimensional and paired). Significance was calculated by comparing data sets with the chemically crosslinked Pichia blank sample.
**Fig. 2****:** Microscopic pictures of NIH3T3 cells on collagen hydrogel coatings. The cells were incubated on the hydrogels for 24 h under culture conditions (37 °C, moisturized air, 5% CO₂). C stands for chemically crosslinked; rCol stands for rat tail collagen.

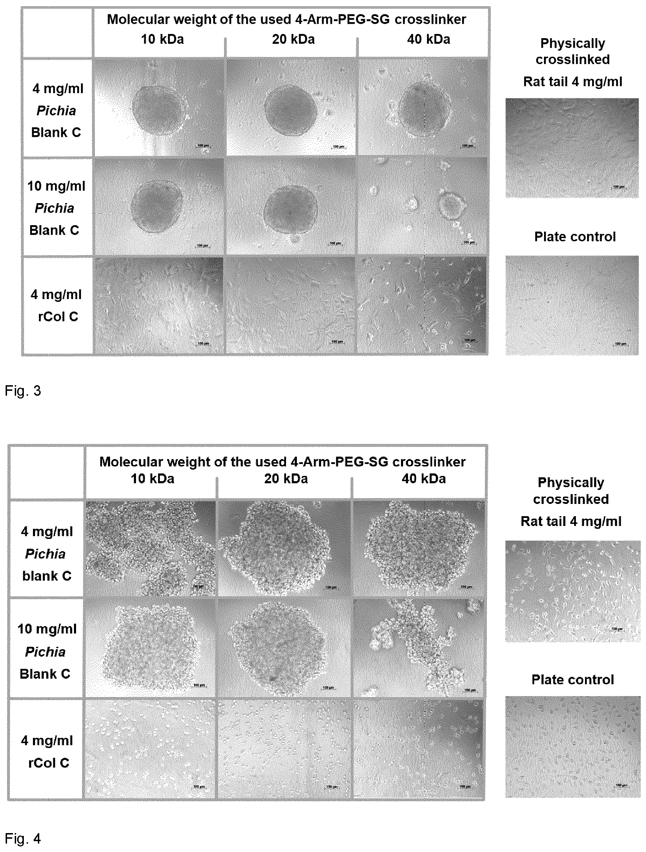

**Fig. 3****:** Microscopic pictures of HFF cells (human fibroblasts) on collagen hydrogel coatings. The cells were incubated on the hydrogels for 24 h under culture conditions (37 °C, moisturized air, 5% CO₂).C stands for chemically crosslinked; rCol stands for rat tail collagen.
**Fig. 4****:** Microscopic pictures of HeLa cells (human epithelial cells) on collagen hydrogel coatings. The cells were incubated on the hydrogels for 24 h under culture conditions (37 °C, moisturized air, 5% CO₂).C stands for chemically crosslinked; rCol stands for rat tail collagen.

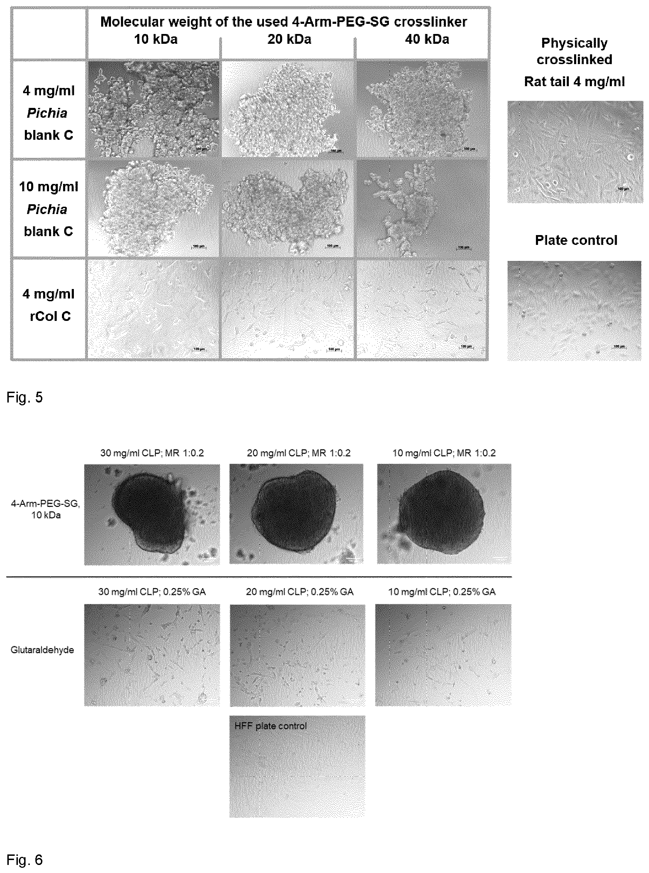

**Fig. 5****:** Microscopic pictures of Cal72 cells (human bone cells) on collagen hydrogel coatings. The cells were incubated on the hydrogels for 24 h under culture conditions (37 °C, moisturized air, 5% CO₂).C stands for chemically crosslinked; rCol stands for rat tail collagen.
**Fig. 6****:** (A) Brightfield imaging after cell adhesion experiment of HFF cells exposed to different hydrogel surfaces according to the present invention for 24 h under culture conditions.

### Detailed description

The present invention refers to a hydrogel, obtained by reacting at least one collagen-like protein and at least one cross-linker comprising at least two groups each comprising a polyalkylene glycol moiety and a succinimidyl group, wherein the at least one bacterial collagen-like protein is a polypeptide that is at least ≥ 60%, identical to the amino acid sequence of SEQ ID NO:1, SEQ ID NO:2, SEQ ID NO:3, SEQ ID NO:4, SEQ ID NO:5, SEQ ID NO:6, SEQ ID NO:7, SEQ ID NO:8 or SEQ ID NO:9.

Furthermore, the present invention refers to the use of the hydrogel according to the present invention as stent, hydrogel for wound sealing, drug delivery hydrogel, hydrogel for healing promotion, anti-adhesion barrier, coating, sponge, or in cell culture, spheroid production, embryoid body and production.

These and other aspects, embodiments, features, and advantages of the invention will become apparent to a person skilled in the art through the study of the following detailed description and claims. Furthermore, it will readily be understood that the examples contained herein are intended to describe and illustrate the invention but not to limit the invention and that, in particular, the invention is not limited to these examples.

Numerical ranges that are indicated in the format "from x to y" also include the stated values. If several preferred numerical ranges are indicated in this format, it is self-evident that all ranges that result from the combination of the various endpoints are also included.

"One or more", as used herein, relates to at least one and comprises 1, 2, 3, 4, 5, 6, 7, 8, 9 or more of the referenced species. Similarly, "at least one" means one or more, i.e. 1, 2, 3, 4, 5, 6, 7, 8, 9 or more. "At least one", as used herein in relation to any component, refers to the number of chemically different molecules, i.e. to the number of different types of the referenced species, but not to the total number of molecules. For example, "at least one cross-linker" means that at least one type of cross-linker falling within the definition for a cross-linker is used but that also two or more different types of cross-linkers falling within this definition can be present, but does not mean that only one or more molecules of one type of cross-linker are present.

All percentages given herein in relation to the compositions or formulations relate to wt.-% relative to the total weight of the respective composition, if not explicitly stated otherwise.

"Essentially free of" according to the present invention with regard to compounds means that the compound can only be present in an amount, which does not influence the characteristics of the composition, in particular the respective compound is present in less than 3 wt.-%, preferably 1 wt.-%, more preferably 0.01 wt.-%, based on the total weight of the composition or is not present at all.

The weight average molecular weight Mw and the number average molecular weight Mn can be determined by GPC employing polystyrene standards or size exclusion chromatography.

A hydrogel is according to the present invention considered to be non-adhesive, if after 2D cell cultivation for 24 h on the hydrogel and subsequent washing (3x 1x PBS) not more than 10%, preferably 5% more preferably 0% of cells remain on the surface. The percentage is calculated by comparing the number of cells after washing on the hydrogel with the number of cells on an optimized cell culture surface (plasma treated PS) also after washing. The hydrogel area and the optimized plate surface were identical as well as the cell number during cell seeding.

To obtain the hydrogel at least one collagen-like protein is reacted with at least one specific crosslinker.

In general, all collagen-like proteins are suitable.

In a preferred embodiment of the present invention the collagen-like protein is a collagen-like protein from *Streptococcus pyogenes,* which is preferably the Scl2 protein from *Streptococcus pyogenes.*

Expression of collagen-like proteins have been attempted in several systems, including *Escherichia coli* and *Saccharomyces cerevisiae.*

In one embodiment the at least one collagen-like protein is a bacterial collagen-like protein, preferably produced by fermentation in *Pichia, Brevibacillus, Bacillus, Escherichia* or *Corynebacterium,* preferably *Pichia pastoris, Brevibacillus choshinensis* or *Corynebacterium glutamicum.*

In a preferred embodiment the collagen-like proteins may be expressed in *Corynebacterium,* preferably in *Corynebacterium glutamicum.*

One particularly suitable collagen-like protein is derivable from a polynucleotide encoding an amino acid sequence that is at least ≥ 60%, identical to the amino acid sequence of SEQ ID NO:1, wherein the polynucleotide is a replicable polynucleotide encoding a collagen-like protein and wherein the amino acid sequence comprises a deletion of at least 38 amino acids at the N-terminus of the amino acid sequence of SEQ ID NO:1.

It is preferred, when the amino acid sequence comprises a deletion of between 38 and 74 amino acids at the N-terminus of the amino acid sequence of SEQ ID NO:1. This includes a complete deletion of the N-terminal V-domain (comprising 74 amino acids) and different truncations of the V-domain of at least 38 amino acids.

The amino acid sequence is at least ≥ 60%, identical to the amino acid sequence of SEQ ID NO:1, SEQ ID NO:2, SEQ ID NO:3 or SEQ ID NO:4, SEQ ID NO:5, SEQ ID NO:6, SEQ ID NO:7, SEQ ID NO:8 or SEQ ID NO:9.

In a further configuration, the amino acid sequence that is at least ≥ 65%, or ≥ 70%, or ≥ 75%, or ≥ 80%, or ≥ 85% identical to the amino acid sequence of SEQ ID NO:2, SEQ ID NO:3 or SEQ ID NO:4, SEQ ID NO:5, SEQ ID NO:6, SEQ ID NO:7, SEQ ID NO:8 or SEQ ID NO:9.

In a preferred configuration, the polynucleotide encodes an amino acid sequence that is at least ≥ 90%, ≥ 92%, ≥ 94%, ≥ 96%, ≥ 97%, ≥ 98%, ≥ 99% or 100%, preferably ≥ 97%, particularly preferably ≥ 98%, very particularly preferably ≥ 99%, and extremely preferably 100%, identical to the amino acid sequence of SEQ ID NO:2, SEQ ID NO:3 or SEQ ID NO:4, SEQ ID NO:5, SEQ ID NO:6, SEQ ID NO:7, SEQ ID NO:8 or SEQ ID NO:9.

In a preferred embodiment of the present invention the polynucleotide is a replicable nucleotide sequence encoding the collagen-like protein from *Streptococcus pyogenes.*

Polynucleotide and nucleic acid molecules comprising such sequences and encoding polypeptide variants of SEQ ID NO:1 to 9, which contain one or more insertion(s) or deletion(s) are suitable as well. Preferably, the polypeptide contains a maximum of 5, a maximum of 4, a maximum of 3, or a maximum of 2, insertions or deletions of amino acids.

Plasmids and vectors that comprise the nucleotide sequences according to the invention and optionally replicate in microorganisms of the genera *Pichia, Corynebacterium, Pseudomonas* or *Escherichia* or are suitable therefor. In a preferred configuration, the vector comprising the nucleotide sequences according to the present invention is suitable for replication in yeast of the genus *Pichia pastoris.*

Microorganisms of the genera *Pichia, Corynebacterium, Pseudomonas* or *Escherichia* that comprise the polynucleotides, vectors and polypeptides according to the invention are suitable as well. Preferred microorganisms are *Pichia pastoris, Brevibacillus choshinensis* or *Corynebacterium glutamicum.*

Microorganism of the species *P. pastoris, E. coli, P. putida* or *C*. *glutamicum* comprising any of the nucleotide sequences according to the present invention any of the polypeptides or any of the vectors according to the present invention are suitable.

The microorganism may be a microorganism in which the nucleotide sequence is present in overexpressed form.

Overexpression according to the invention means, generally, an increase in the intracellular concentration or activity of a ribonucleic acid, a protein (polypeptide) or an enzyme, compared with the starting strain (parent strain) or wild-type strain, if this is the starting strain. A starting strain (parent strain) is taken to mean the strain on which the measure leading to the overexpression was carried out.

In the overexpression, the methods of recombinant overexpression are preferred. These include all methods in which a microorganism is produced using a DNA molecule provided *in vitro.* Such DNA molecules comprise, for example, promoters, expression cassettes, genes, alleles, encoding regions etc. These are converted into the desired microorganism by methods of transformation, conjugation, transduction or like methods.

The extent of the expression or overexpression can be established by measuring the amount of the mRNA transcribed by the gene, by determining the amount of the polypeptide, and by determining the enzyme activity.

The bacterial collagen-like protein can be obtained in a fermentative process comprising the following steps:
a) fermentation of a microorganism according to the present invention in a medium,
b) accumulation of the bacterial collagen-like protein in the medium, wherein a fermentation broth is obtained.

The culture medium or fermentation medium that is to be used must appropriately satisfy the demands of the respective strains. Descriptions of culture media of various microorganisms are contained in the handbook "Manual of Methods for General Bacteriology" of the American Society for Bacteriology (Washington D.C., USA, 1981). The terms culture medium and fermentation medium or medium are mutually exchangeable.

As carbon source, sugars and carbohydrates can be used, such as, e.g., glucose, sucrose, lactose, fructose, maltose, molasses, sucrose-containing solutions from beet sugar or sugar cane processing, starch, starch hydrolysate and cellulose, oils and fats, such as, for example, soybean oil, sunflower oil, groundnut oil and coconut fat, fatty acids, such as, for example, palmitic acid, stearic acid and linoleic acid, alcohols such as, for example, glycerol, methanol and ethanol, and organic acids, such as, for example, acetic acid or lactic acid.

As nitrogen source, organic nitrogen compounds such as peptones, yeast extract, meat extract, malt extract, corn-steep liquor, soybean meal and urea or inorganic compounds such as ammonium sulphate, ammonium chloride, ammonium phosphate, ammonium carbonate and ammonium nitrate can be used. The nitrogen sources can be used individually or as a mixture.

As phosphorus source, phosphoric acid, potassium dihydrogenphosphate or dipotassium hydrogenphosphate or the corresponding sodium-containing salts can be used.

The culture medium must, in addition, contain salts, for example in the form of chlorides or sulphates of metals such as, for example, sodium, potassium, magnesium, calcium and iron, such as, for example, magnesium sulphate or iron sulphate, which are necessary for growth. Finally, essential growth substances such as amino acids, for example homoserine and vitamins, for example thiamine, biotin or pantothenic acid, can be used in addition to the above-mentioned substances.

Said starting materials can be added to the culture in the form of a single batch or supplied in a suitable manner during the culturing.

Basic compounds such as sodium hydroxide, potassium hydroxide, ammonia or ammonia water, or acid compounds such as phosphoric acid or sulphuric acid, are used in a suitable manner for pH control of the culture. The pH is generally adjusted to 6.0 to 8.5, preferably 6.5 to 8. For control of foam development, antifoams can be used, such as, for example, polyglycol esters of fatty acids. For maintaining the stability of plasmids, suitable selectively acting substances such as, for example, antibiotics, can be added to the medium. The fermentation is preferably carried out under aerobic conditions. In order to maintain said aerobic conditions, oxygen or oxygen-containing gas mixtures such as, for example, air, are introduced into the culture. The use of liquids that are enriched with hydrogen peroxide is likewise possible. Optionally, the fermentation is carried out at superatmospheric pressure, for example at a superatmospheric pressure of 0.03 to 0.2 MPa. The temperature of the culture is usually 20°C to 45°C, and preferably 25°C to 40°C, particularly preferably 30°C to 37°C. In the case of batch or fed-batch processes, the culturing is preferably continued until an amount sufficient for the measure of obtaining the desired organic chemical compound has formed. This goal is usually reached within 10 hours to 160 hours. In continuous processes, longer culture times are possible. Owing to the activity of the microorganisms, enrichment (accumulation) of the fine chemicals in the fermentation medium and/or in the cells of the microorganisms occurs.

Examples of suitable fermentation media may be found, inter alia, in patent documents US 5,770,409, US 5,990,350, US 5,275,940, WO 2007/012078, US 5,827,698, WO 2009/043803, US 5,756,345 or US 7,138,266; appropriate modifications may optionally be carried out to the requirements of the strains used.

The process may be characterized in that it is a process which is selected from the group consisting of batch process, fed-batch process, repetitive fed-batch process and continuous process.

The process may be further characterized in that the fine chemical or a liquid or solid fine chemical-containing product is obtained from the fine chemical-containing fermentation broth.

The performance of the processes or fermentation processes according to the invention with respect to one or more of the parameters selected from the group of concentration (compound formed per volume), yield (compound formed per carbon source consumed), volumetric productivity (compound formed per volume and time) and biomass-specific productivity (compound formed per cell dry mass or bio dry mass and time or compound formed per cell protein and time) or other process parameters and combinations thereof, is increased by at least 0.5%, at least 1%, at least 1.5% or at least 2%, based on processes or fermentation processes with microorganisms in which the promoter variant according to the invention is present.

Owing to the measures of the fermentation, a fermentation broth is obtained which contains the desired collagen-like protein, and preferably amino acid or organic acid.

Then, a product in liquid or solid form that contains the collagen-like protein is provided or produced or obtained.

A fermentation broth means, in a preferred embodiment, a fermentation medium or nutrient medium in which a microorganism was cultured for a certain time and at a certain temperature. The fermentation medium, or the media used during the fermentation, contains/contain all substances or components that ensure production of the desired collagen-like protein and typically ensure growth and/or viability.

On completion of the fermentation, the resultant fermentation broth accordingly contains
a) the biomass (cell mass) of the microorganism resulting from growth of the cells of the microorganism,
b) the desired collagen-like protein formed in the course of the fermentation,
c) the organic by-products possibly formed in the course of the fermentation, and
d) the components of the fermentation medium used, or of the starting materials, that are not consumed by the fermentation, such as, for example, vitamins such as biotin, or salts such as magnesium sulphate.

The organic by-products include substances which are generated in addition to the respective desired compound by the microorganisms used in the fermentation and are possibly secreted.

The fermentation broth is withdrawn from the culture vessel or the fermentation container, optionally collected, and used for providing a product in liquid or solid form containing the collagen-like protein. The expression "obtaining the collagen-like protein-containing product" is also used therefor. In the simplest case, the collagen-like protein-containing fermentation broth withdrawn from the fermentation container is itself the product obtained.

By way of one or more of the measures selected from the group
a) partial (> 0% to < 80%) to complete (100%) or virtually complete (≥ 80%, ≥ 90%, ≥ 95%, ≥ 96%, ≥ 97%, ≥ 98%, ≥ 99%) removal of the water,
b) partial (> 0% to < 80%) to complete (100%) or virtually complete (≥ 80%, ≥ 90%, ≥ 95%, ≥ 96%, ≥ 97%, ≥ 98%, ≥ 99%) removal of the biomass, wherein this is optionally inactivated before the removal,
c) partial (> 0% to < 80%) to complete (100%) or virtually complete (≥ 80%, ≥ 90%, ≥ 95%, ≥ 96%, ≥ 97%, ≥ 98%, ≥ 99%, ≥ 99.3%, ≥ 99.7%) removal of the organic by-products formed in the course of the fermentation, and
d) partial (> 0%) to complete (100%) or virtually complete (≥ 80%, ≥ 90%, ≥ 95%, ≥ 96%, ≥ 97%, ≥ 98%, ≥ 99%, ≥ 99.3%, ≥ 99.7%) removal of the components of the fermentation medium used or the starting materials that are not consumed by the fermentation,
a concentration or purification of the desired collagen-like protein is achieved from the fermentation broth. In this manner, products are isolated that have a desired content of the compound.

The partial (> 0% to < 80%) to complete (100%) or virtually complete (≥ 80% to < 100%) removal of the water (measure a)) is also termed drying.

In a variant of the process, by complete or virtually complete removal of the water, the biomass, the organic by-products and the non-consumed components of the fermentation medium used, pure (≥ 80% by weight, ≥ 90% by weight) or high-purity (≥ 95% by weight, ≥ 97% by weight, ≥ 99% by weight) product forms of the desired collagen-like protein, preferably bacterial collagen-like protein, are successfully arrived at. For the measures according to a), b), c) or d), a great variety of technical instructions are available in the prior art.

In the case of processes for producing bacterial collagen-like protein processes are preferred in which products are obtained that do not contain any components of the fermentation broth. These products are used, in particular, in human medicine, in the pharmaceuticals industry, and in the food industry.

The at least one cross-linker comprises at least two, preferably two to six, more preferably two to four, most preferably four, groups each comprising a polyalkylene glycol moiety, preferably polyethylene glycol moiety or polypropylene glycol moiety, more preferably polyethylene glycol moiety, and a succinimidyl group.

In a preferred embodiment the cross-linker has one of following formulae (I) or (II): 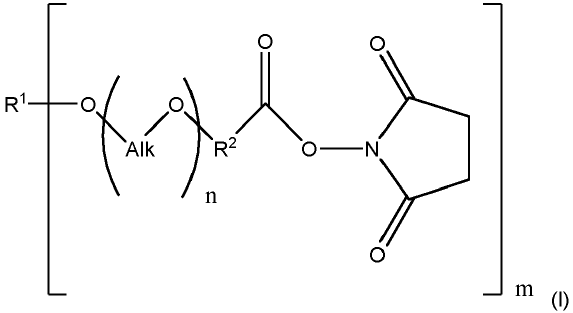 wherein
R¹ is a linear or branched alkyl group having up to 12 carbon atoms, preferably up to 8 carbon atoms, more preferably having five carbon atoms;
Alk is -CH₂-, -CH₂-CH₂- or -CH₂-CH₂-CH₂-, preferably -CH₂-CH₂-;
n is an integer from 1 to 1350, preferably, 50 to 1000, more preferably 125 to 660;
R² is -CH₂-, -C₂H₄-NH-(C=O)-C₃H₆-, -C₂H₄-O-, -C₂H₄-O-(C=O)-C₃H₆-, -C₂H₄-NH-(C=O)-C₂H₄- or -C₂H₄-O-(C=O)-C₂H₄-;
m is an integer from 2 to 8, preferably 4 to 8, more preferably 4;
   or 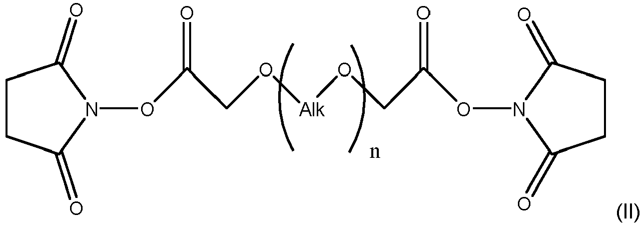 wherein
Alk is -CH₂-, -CH₂-CH₂- or -CH₂-CH₂-CH₂-, preferably -CH₂-CH₂-; and
n is an integer from 1 to 1350, preferably 50 to 1000, more preferably 125 to 660.

In one embodiment the cross-linker has following formula (III) 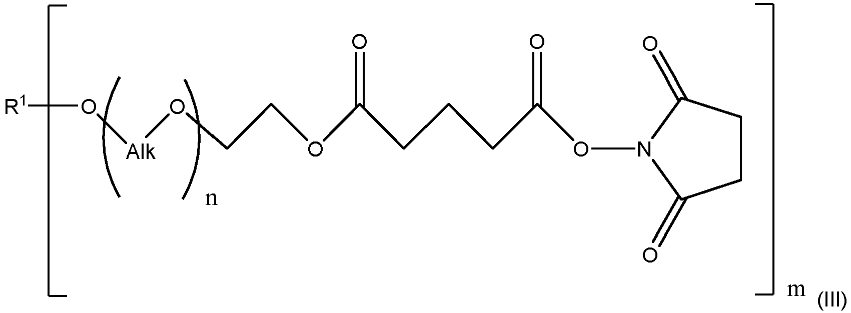 wherein
R¹ is a linear or branched alkyl group having up to 12 carbon atoms, preferably up to 8 carbon atoms, more preferably having five carbon atoms, most preferably is 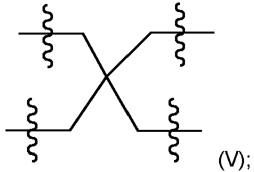
Alk is -CH₂-, -CH₂-CH₂- or -CH₂-CH₂-CH₂-, preferably -CH₂-CH₂-;
n is an integer from 1 to 1350, preferably, 50 to 1000, more preferably 125 to 660;
m is an integer from 2 to 8, preferably 4 to 8, most preferably 4.

In one embodiment the cross-linker has a molecular weight of 2.000 to 40.000 g/mol.

In one embodiment the reaction takes place in an aqueous medium, preferably a buffer, more preferably 2-(4-(2-Hydroxyethyl)-1-piperazinyl)-ethansulfonic acid(HEPES), having a pH value of 6 to 8, preferably 7 to 8, more preferably 8.

In one embodiment the collagen-like protein concentration in the hydrogel is 1 to 100 mg/ml, preferably 2.5 - 40 mg/ml.

In one embodiment the molar ratio of lysine groups in the collagen-like protein to the succinimidyl groups in the cross-linker is 1:0.01 to 1:1.6.

In one embodiment the hydrogel is non-adhesive.

In one embodiment the hydrogel has anti-fouling properties.

### Examples: Materials & Methods

### I. Production of collagen-like protein

The bacterial collagen-like protein was produced in different host cells by fermentation.

The collagen-like protein was produced in the yeast host cell *Pichia pastoris* by fermentation. To produce Scl2 from *Streptococcus pyogenes* in *Pichia pastoris,* the sequence of the collagen-like protein (full-length protein and truncated variants and no-V-domain variant), has been codon optimized using different algorithms, and cloned in a secretion vector for *Pichia pastoris.* The sequences used are summarized in SEQ ID NO:1 to SEQ ID NO:9. For each of the specific sequences, a vector was transformed in *Pichia pastoris* following standard protocol and a standard expression protocol in fed-batch mode was applied *(*Damasceno, L.M., Huang, CJ. & Batt, C.A. Protein secretion in Pichia pastoris and advances in protein production. Appl Microbiol Biotechnol 93, 31-39 (2012*)).* The collagen domain of the Scl2p protein was detected via HPLC analysis in the supernatant of cell culture. Upon fermentation, supernatant has been separated from biomass via centrifugation (12000g, 5 mins at room temperature).

The collagen domain of the Scl2p protein based on the sequences SEQ ID NO:1 to SEQ ID NO:9 could be produced under similar conditions using either *E*. *coli, B. choshinensis* or *C*. *glutamicum.* In case of a production in yeast or *C*. *glutamicum,* the collagen domain is secreted by the cell. No cell lysis is needed as an initial purification step in this approach. In case of a production in *E*. *coli a* cell lysis is mandatory to remove the collagen domain from the cell.

The full-length collagen-like protein, a truncated variant (truncation 3) and the no-V-domain variant (based on the gene *scl2* from *Streptococcus pyogenes*) were also expressed in *Brevibacillus choshinensis.* Therefore, the corresponding DNA sequences were cloned into a suitable secretion vector for B. *choshinensis.* Transformation of B. *choshinensis* with the new constructed plasmids was done according to Mizukami et al. 2010 (Curr Pharm Biotechnol 2010, 13:151-258).

The B. *choshinensis* strains were analyzed for their ability to produce the different collagen-like proteins in batch cultivations at 33°C and pH 7 using the DASGIP^{®} parallel bioreactor system from Eppendorf (Hamburg, Germany). The fermentation was performed using 1 L reactors. The production medium (TM medium, Biomed Res Int 2017, 2017: 5479762) contained 10 g/L glucose. Upon fermentation, supernatant has been separated from biomass by centrifugation and was used for SDS PAGE analysis. For all three variants, collagen domain of the Scl2p protein was produced.

The full-length collagen-like protein and the no-V-domain variant (based on the gene *scl2* from *Streptococcus pyogenes*) were also expressed in *Corynebacterium glutamicum.* Therefore, the corresponding DNA sequences were cloned together with an upstream located signal peptide for protein secretion into a shuttle vector for C. *glutamicum* (Biotechnology Techniques 1999, 13: 437-441.). The *C. glutamicum* strain ATCC 13032 was transformed with the new constructed plasmids by means of electroporation as described by Ruan et al. (Biotechnology Letters 2015, 37: 2445-2452).

The *C. glutamicum* strains were analyzed for their ability to produce the different collagen proteins in fed-batch cultivations at 30°C and pH 7 using the DASGIP^{®} parallel bioreactor system from Eppendorf (Hamburg, Germany). The fermentation was performed using 1 L reactors. The production medium contained 20 g/L glucose in the batch phase and the fed-batch phase was run with a glucose feed of 4 g/L*h. Upon fermentation, supernatant has been separated from biomass by centrifugation and was used for HPLC analysis. For both variants, collagen domain of the Scl2p protein was produced. For the truncated variant of the collagen-like protein, titer was higher as for the full-length variant.

After cell separation (via centrifugation) and folding of the bacterial collagen-like protein (via cooling of the concentrate) the bacterial collagen-like protein was purified using precipitation with 2-Propanol at 15 v%. After precipitation of the Scl2 protein a centrifugation was performed. The pellet was dissolved in water, the triple helical Scl2 protein was unfolded at 40°C and filtered through a 100 kD membrane. This step serves to remove large sized impurities. The collected permeate was then concentrated in the consecutive 10 kD filtration. The retentate was washed to remove small sized impurities.

By that means a triple helical Scl2 protein purity >75 w% was achieved.

### II. Hydrogel Synthesis

**Test samples:** This protocol describes the hydrogel preparation for non-adhesive collagen hydrogels. The following materials are required:
a. 1 M HEPES buffer pH 8.0 in ddH₂O
b. 40 mg/ml collagen-like protein solution in ddH₂O which is prepared by dissolving 40 mg collagen-like peptide in a sterile tube and 1 ml sterile ddH₂O is added on top. The mixture is prepared one day in advance to ensure complete dissolution & placed overnight on an orbital shaker (200 rpm) at RT.
c. 192.61 mg/ml 4-Arm-PEG-SG in ddH₂O with 10 kDa (JenKem Technology USA)
d. ddH₂O

The needed final collagen-like protein concentration and the final molar ratio of the crosslinker determine the needed volume of each solution. The stock solution of 1 M HEPES pH 8.0 buffer is diluted 1:10 for a final buffer concentration of 100 mM.

The hydrogel formulations were prepared in polypropylene tubes and subsequently they were filled in the final vessels using reverse pipetting to avoid bubble formation. The components were added in the following order: ddH₂O, buffer, collagen and crosslinker. Before adding the crosslinker, the formulation is homogenized by mixing. If bubble entry occurs, use centrifugation to remove bubbles. Subsequently, the linker was added, and the mixture was homogenized again. To remove bubbles occurring from mixing, briefly spinning down with a centrifuge can remove the bubbles. If not stated otherwise, the samples were incubated overnight (12-24 h) at RT (25 °C) under humid conditions to avoid hydrogel drying.

**Control samples:** Control samples were made from rat tail collagen (Sigma & Aldrich, article number: C7661). Additionally, the following materials are needed:
a. 10x PBS buffer
b. 1 M NaOH
c. 0.1% CH₃COOH in ddH₂O
d. 0.04% Bromothymol blue (BTB) in ddH₂O
e. Multipette (Eppendorf)
f. 1 M HEPES buffer pH 8.0
g. ddH₂O

Rat tail collagen is dissolved in 0.1% CH₃COOH 2 days in advance with a concentration of 6 mg/ml to ensure a homogenous solution. To transfer the highly viscous material, a Multipette (Eppendorf) was used.
Physically crosslinked rat tail collagen samples: 10x PBS solution was diluted 1:10 to yield 1x PBS. Collagen is diluted to its desired final concentration by using 10x PBS, ddH₂Oand 1 M NaOH. Enough NaOH was added to reach a pH of 7.2 - 7.4.
The compound solutions were cooled (to slow down the gelation process) in advance for 15 min on ice. The compounds were added in the following order: ddH₂O, collagen (then mixing), 10x PBS (then mixing), 1 M NaOH (then mixing). Then mix thoroughly again and spin sample down by centrifugation to remove any bubbles. Transfer collagen solution to vessels, using the Multipette and incubate the plate at 37 °C under humid conditions for 1 h.
Chemically crosslinked rat tail collagen sample: When the crosslinker is added directly to the highly viscous rat tail collagen solution, instant gelation can occur. Therefore, the rat tail collagen was prepared identical to the physical crosslinking protocol stated above with the needed final collagen concentrations.
Then, 2x the final crosslinker concentration was dissolved in 100 mM HEPES buffer pH 8.0 in the same hydrogel volume which was applied to the final vessel for gelation. Here, the crosslinking solution remained 2 h on top of the physically crosslinked hydrogel at room temperature (25°C) under humid conditions. Afterwards the residual supernatant was removed and the hydrogel was washed 3x with 2x PBS buffer.

### III. Hydrogel washing

Hydrogels were topped with respectively 150 µl (96 well plate format) or 300 µl (48 well plate format) pure DMEM medium without serum (FBS = fetal bovine serum) for 1 h at RT under orbital shaking each (200 rpm). Then the washing solution was exchanged. The washing procedure was repeated 3× in total.

### IV. Cell Cultivation

Standard conditions were used comprising: 37°C, 5% CO₂ and a humid atmosphere.

### Experiment I: Cell Adhesion with subsequent calcein-labelling

Evonik Collagen hydrogel samples were prepared with a volume height of 2 mm in two black 96 well plates for fluorescence measurements (≙ 64 µl/well). Controls: chemically and physically crosslinked rat tail collagen and the culture-optimized plate culture. The samples were prepared as indicated above with 3 and 4 mg/ml collagen in triplicate (similar in both plates).

For chemical crosslinking, 4-Arm PEG Succinimidyl Glutarate (4-Arm-PEG-SG, 10 kDa, article number: A7031-1 / 4ARM-SG-10K) was used with a molar ratio of 1:0.8 where 1 represents the lysine side chains in the collagen-like protein and 0.8 the NHS groups in the crosslinker. A pipetting scheme for this experiment is attached. Empty wells were filled with water to create humid conditions to reduce hydrogel drying. The plates were sealed with parafilm and incubated for 4 h at 25 °C (RT). Then, the plates were stored overnight (12-24 h) at 37 °C und humid conditions. Afterwards, the hydrogels were washed 3× as indicated above.

For this experiment, the "Vybrant^{™} Cell Adhesion Assay Kit" (supplier: ThermoFisher, article number: V13181) was applied. Annealing to the manual, the following steps were applied:
a.) Background signal: The hydrogels background signal was recorded. Therefore, superficial liquid (on top of the hydrogels) was removed and the fluorescence emission was recorded with an MTP reader (TecanReader) (Settings: Fluorescence read-out; Top reading function; Z-focus 14600 - Ex. 490 nm, Em. 522 nm + Multiple measurement option.
b.) Staining & Seeding: Prepare a cell suspension of 1×10⁶ NIH3T3 mouse fibroblasts/ml medium. Label cells with 5 µl of 1 mM calcein-AM solution per 1 ml cell suspension for 30 min at 37 °C (kit contains staining solution). Wash the cells twice with serum-free DMEM medium. Seed respectively 100 µl stained cell suspension (1×10⁶ cells/ml) per well in both black plates. For control samples, seed on plate surface too. Incubate the cells under standard conditions for 2 h.
c.) Washing: Wash one plate 4× with respectively 150 µl serum-free medium. Replace liquid carefully in both plates by 200 µl 1× PBS.
d.) Signal detection: Read out the fluorescence emission with the same settings as for the background signal.
e.) Microscopic pictures: Record fluorescence emission pictures for all samples.

Cells on collagen-like hydrogels showed cell aggregation and spheroid formation, while cells on the culture plate's surface as well as on the rat tail collagen hydrogels adhered to the hydrogel surfaces.

**Table 1: Pipetting scheme for chemically crosslinked collagen formulations. Depicted is a Mastermix for 750 µl.**

| **final concentration** | | | **stock concentration** | | **volume (stock solution)** | | | |
|---|---|---|---|---|---|---|---|---|
| **Collagen** | **molar ratio** | **4-Arm-PEG-NHS, 10 kDa** | **Collagen** | **4-Arm-PEG-NHS, 10 kDa** | **Collagen** | **4-Arm-PEG-NHS, 10 kDa** | **0.1 M HEPES pH=8.0** | **ddH2O** |
| **mg/mL** | | **mg/mL** | **mg/mL** | **mg/mL** | **µl** | **µl** | **µl** | **µl** |
| 2 | 0.8 | 3.84 | 20 | 192.16 | 75 | 15.0 | 75 | 585 |
| 3 | 0.8 | 5.76 | 20 | 192.16 | 112.5 | 22.5 | 75 | 540 |
| 4 | 0.8 | 7.68 | 20 | 192.16 | 150 | 30.0 | 75 | 495 |

### Experiment II: Cell Adhesion with subsequent LIVE/DEAD or DAPI/Phalloidin staining

Hydrogel samples were made from collagen-like protein as well as physically and chemically crosslinked rat tail collagen with a height of 2 mm as described above in transparent 48 well plates (≙ 220 µl/well). The hydrogels were washed as indicated above and then NIH3T3 fibroblasts were seeded on top with respectively 3×10⁴ cells/well by applying 300 µl of a 1×10⁵ cells/ml suspension per well. After 1 d cultivation under standard conditions, the cells were stained the following:
I) LIVE/DEAD staining: The "LIVE/DEAD^{™} Viability/Cytotoxicity Kit, for mammalian cells" from ThermoFisher was used (article number: L3224) according to the supplier's manual description with the following parameters: 2.5 µl 4 mM calcein-AM solution and 5 µl 2 mM ethidium homodimer-1 solution were added to 5 ml 1×PBS. Additionally, 1 µl Hoechst 33324 (Trihydrochloride, Trihydrate - 10 mg/mL Solution in Water; ThermoFisher; catalog number: H3570) was added. The homogenized solution was added to the cells with respectively 300 µl/well and fluorescence emission pictures were recorded after 30 min incubation time under standard culture conditions.
II) Hoechst/Phalloidin staining:
   a. Washing: Remove medium and wash cells twice with 1× PBS.
   b. Fixation: Cells were fixated by incubation with 4% aqueous paraformaldehyde solution for 15 min.
   c. Washing: Cells were washed once with 1× PBS
   d. Permeabilization: Expose cells to 0.1% Triton X-100 in PBS (10 µl Triton X-100 in 10 ml PBS)
   e. Staining: 25 µl Alexa Fluor^{™} 488 Phalloidin (ThermoFisher, catalog number: A12379) was diluted in 1 ml PBS/BSA solution (1× PBS containing 1% BSA). 200 µl staining solution was applied per sample for 20 min incubation time at RT (25 °C). Cover plate; keep it in the dark.
   f. Washing: Remove staining solution and wash twice with respectively 500 µl PBS
   g. Nuclei staining: Cover the stained cells with 200 µl of Hoechst staining solution containing 1 µl Hoechst 33342 (Trihydrochloride, Trihydrate - 10 mg/mL Solution in Water; ThermoFisher; catalog number: H3570) in 5 ml 1× PBS.
   h. Pictures: After staining, fluorescence emission pictures were recorded using a microscope.

Cells on collagen-like hydrogels showed cell aggregation and spheroid formation, while cells on the culture plate's surface as well as on the rat tail collagen hydrogels adhered to the hydrogel surfaces

### Experiment III: Different cell lines (Cal72, 3T3 and HFF cells) and Mw of the crosslinker

Shown cell repellant properties with NIH3T3 fibroblasts (Experiment I and II) shall be validated. Additionally, cell repellant properties for other cell types including bone cells (Cal72), human fibroblasts (HFF) and human epithelial cells (HeLa) shall be tested. Controls: chemically and physically crosslinked rat tail collagen hydrogels were tested as well as the optimized plate surface of the used 96 well culture plate. Molar ratios of the crosslinker were used identical to experiment I (1:0.8) as well as the sample preparations including synthesis and washing. The experiment was performed in a 96 well plate format. 1×10⁴ cells/well were used (100 µl of a 1×10⁵ cells/ml stock solution). All collagen samples were prepared with finally 4 mg/ml. Additionally, hydrogels with 10 mg/ml collagen-like protein were used.

In this experiment not only the influence of the cell type but also the linker size was evaluated. Therefore, different molecular weights were used including:
- 4-Arm-PEG-SG, 10 kDa (JenKem Technology, USA; article number: A7031-1)
- 4-Arm-PEG-SG, 20 kDa (JenKem Technology, USA; article number: A7010-1)
- 4-Arm-PEG-SG, 40 kDa (JenKem Technology, USA; article number: *A7017-1)

After 24 h exposure to the hydrogel surfaces, microscopic brightfield pictures were recorded.

The data showed cell repellent properties of the collagen-like hydrogel surface for all tested cell types and the effect was observed with all tested molecular weights of the crosslinker used for this experiment. In comparison, the chemically and physically crosslinked rat tail collagen surfaces as well as the culture plate's surface showed cell adhesion.

### Cell Adhesion

A collagen-like protein from *Streptococcus pyogenes* was added into a sterile 48 well plate (220 µl/well, equals 2 mm hydrogel thickness) using reverse pipetting to avoid bubble formation. The plate was covered to avoid hydrogel drying followed by incubation at 20 - 25 °C overnight. The next day, hydrogels were washed three times with twice the hydrogel volume per well (440 µl) using 1x PBS at room temperature by placing the plate on an orbital shaker (300 rpm). Subsequently, the hydrogels were washed once with 440 µl serum-free culture medium (the removal of the washing solution, HFF cells were added (300 µl of a 1×105 cells/ml suspension per well). The 48 well plate was incubated under culture conditions for 24 h (37 °C, 5% CO₂, humid atmosphere). Then, the cell morphology was visualized using a transmission light microscope and pictures were taken.

Initially, similar molar ratios (MR) were chosen for both crosslinkers. Using a MR of 1:0.2 was sufficient for the PEG linker but formulations containing glutaraldehyde (GA) on the other hand didn't gel. Therefore, the glutaraldehyde concentration was increased (0.25%). This concentration was chosen because previous experiments showed successful gelation with it.

After incubation, expected cell agglomeration (spheroid formation) was observed with the PEG crosslinked hydrogels while cell attachment was overserved using the GA linker. Both linkers react with the same primary amines present in lysine side chains of compositions according to the present invention. This result indicates that cell attachment is orchestrated by the chosen crosslinker.

### Protein sequences

| | |
|---|---|
| SEQ ID NO:1 | *Streptococcus pyogenes* Collagen-like protein (CLP), full length protein |
| SEQ ID NO:2 | *Streptococcus pyogenes* CLP, truncation 3 |
| SEQ ID NO:3 | *Streptococcus pyogenes* CLP, truncation 5 |
| SEQ ID NO:4 | *Streptococcus pyogenes* CLP, no V-domain |
| SEQ ID NO:5 | *Streptococcus pyogenes* CLP, truncation 5 (AGPR mutant) |
| SEQ ID NO:6 | *Streptococcus pyogenes* CLP, truncation 5 (QGPR mutant) |
| SEQ ID NO:7 | *Streptococcus pyogenes* CLP, truncation 5 (VGPA mutant) |
| SEQ ID NO:8 | *Streptococcus pyogenes* CLP, truncation 5 (SGPR mutant) |
| SEQ ID NO:9 | *Streptococcus pyogenes* CLP, truncation 5 (VGPK mutant) |

## Claims

1. Hydrogel obtained by reacting at least one collagen-like protein and at least one crosslinker comprising at least two groups each comprising a polyalkylene glycol moiety and a succinimidyl group, wherein the at least one bacterial collagen-like protein is a polypeptide that is at least ≥ 60%, identical to the amino acid sequence of SEQ ID NO:1, SEQ ID NO:2, SEQ ID NO:3, SEQ ID NO:4, SEQ ID NO:5, SEQ ID NO:6, SEQ ID NO:7, SEQ ID NO:8 or SEQ ID NO:9.

2. Hydrogel according to claim 1, wherein the at least one collagen-like protein is a bacterial collagen-like protein.

3. Hydrogel according to claim 1 or 2, wherein bacterial collagen-like protein is a collagen-like protein from *Streptococcus pyogenes,* preferably Scl2.

4. Hydrogel according to any of the preceding claims, wherein the bacterial collagen-like protein is produced by fermentation in a host cell, wherein the host cell is a yeast cell, preferably *Pichia pastoris* or a bacterial cell, preferably *E*. *coli, Corynebacterium* or *Brevibacterium.*

5. Hydrogel according to any of the preceding claims, wherein the cross-linker has one of following formulae (I) or (II): 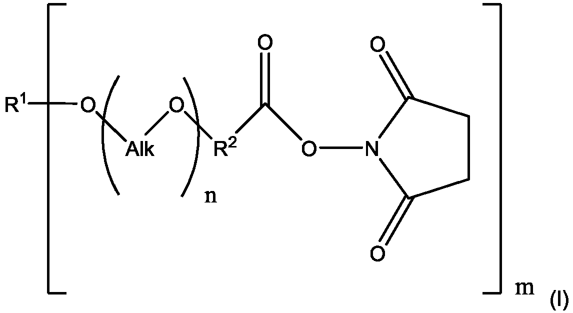 wherein
R¹ is a linear or branched alkyl group having up to 12 carbon atoms;
Alk is -CH₂-, -CH₂-CH₂- or -CH₂-CH₂-CH₂;
n is an integer from 1 to 1350;
R² is -CH₂-, -C₂H₄-NH-(C=O)-C₃H₆-, -C₂H₄-O-, -C₂H₄-O-(C=O)-C₃H₆-, -C₂H₄-NH-(C=O)-C₂H₄- or -C₂H₄-O-(C=O)-C₂H₄-;
m is an integer from 2 to 8;
or 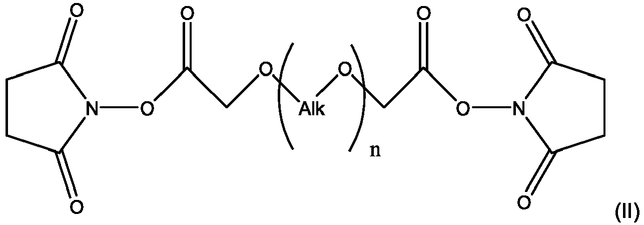 wherein
Alk is -CH₂-, -CH₂-CH₂- or -CH₂-CH₂-CH₂-; and
n is an integer from 1 to 1350.

6. Hydrogel according to any of the preceding claims, wherein the reaction takes place in an aqueous medium having a pH value of 6 to 8.

7. Hydrogel according to any of the preceding claims, wherein the collagen-like protein concentration is 1 to 100 mg/ml.

8. Hydrogel according to any of the preceding claims, wherein the molar ratio of lysine groups in the collagen-like protein to the succinimidyl groups in the cross-linker is 1:0.01 to 1:1.6.

9. Hydrogel according to any of the preceding claims, wherein the hydrogel is non-adhesive.

10. The hydrogel according to any of claims 1 to 9 for use as stent, for wound sealing, drug delivery, healing promotion, as anti-adhesion barrier, coating, sponge, antibacterial dressing, or for use in cell culture, spheroid production and embryoid body formation.

## Patentansprüche

1. Hydrogel, das durch Umsetzen mindestens eines kollagenähnlichen Proteins und mindestens eines Vernetzers erhalten wurde, umfassend mindestens zwei Gruppen, die jeweils eine Polyalkylenglykoleinheit und eine Succinimidylgruppe umfassen, wobei das mindestens eine bakterielle kollagenähnliche Protein ein Polypeptid ist, das zu mindestens ≥ 60 % mit der Aminosäuresequenz von SEQ ID NO:1, SEQ ID NO:2, SEQ ID NO:3, SEQ ID NO:4, SEQ ID NO:5, SEQ ID NO:6, SEQ ID NO:7, SEQ ID NO:8 oder SEQ ID NO:9 identisch ist.

2. Hydrogel nach Anspruch 1, wobei das mindestens eine kollagenähnliche Protein ein bakterielles kollagenähnliches Protein ist.

3. Hydrogel nach Anspruch 1 oder 2, wobei das bakterielle kollagenähnliche Protein ein kollagenähnliches Protein von *Streptococcus pyogenes,* vorzugsweise Scl2, ist.

4. Hydrogel nach einem der vorhergehenden Ansprüche, wobei das bakterielle kollagenähnliche Protein durch Fermentation in einer Wirtszelle hergestellt wird, wobei die Wirtszelle eine Hefezelle, vorzugsweise *Pichia pastoris,* oder eine Bakterienzelle, vorzugsweise *E. coli, Corynebacterium* oder *Brevibacterium,* ist.

5. Hydrogel nach einem der vorhergehenden Ansprüche, wobei der Vernetzer eine der folgenden Formeln (I) oder (II) hat: 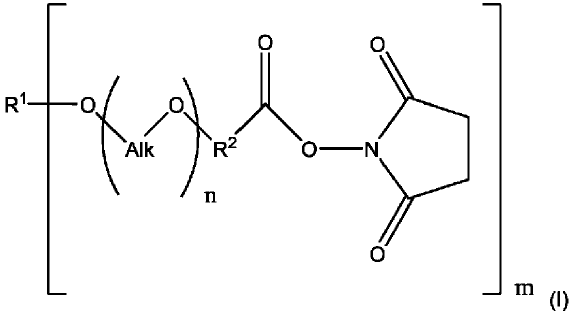 wobei
R¹ eine gerade oder verzweigte Alkylgruppe mit bis zu 12 Kohlenstoffatomen ist,
Alk für -CH₂-, -CH₂-CH₂- oder -CH₂-CH₂-CH₂ steht,
n eine ganze Zahl von 1 bis 1350 ist,
R² für -CH₂-, -C₂H₄-NH- (C=O) -C₃H₆-, -C₂H₄-O-, -C₂H₄-O- (C=O) - C₃H₆-,
-C₂H₄-NH-(C=O)-C₂H₄- oder -C₂H₄-O-(C=O)-C₂H₄- steht,
m eine ganze Zahl von 2 bis 8 ist
oder 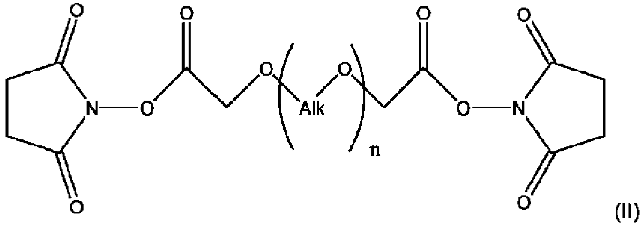
wobei
Alk für -CH₂-, -CH₂-CH₂- oder -CH₂-CH₂-CH₂- steht und
n eine ganze Zahl von 1 bis 1350 ist.

6. Hydrogel nach einem der vorhergehenden Ansprüche, wobei die Umsetzung in einem wässrigen Medium mit einem pH-Wert von 6 bis 8 erfolgt.

7. Hydrogel nach einem der vorhergehenden Ansprüche, wobei die Konzentration des kollagenähnlichen Proteins 1 bis 100 mg/ml beträgt.

8. Hydrogel nach einem der vorhergehenden Ansprüche, wobei das Molverhältnis von Lysingruppen im kollagenähnlichen Protein zu den Succinimidylgruppen im Vernetzer 1:0,01 bis 1:1,6 beträgt.

9. Hydrogel nach einem der vorhergehenden Ansprüche, wobei das Hydrogel nicht-haftend ist.

10. Hydrogel nach einem der Ansprüche 1 bis 9 zur Verwendung als Stent, zur Wundversiegelung, Arzneimittelzuführung, Heilungsförderung, als Antihaftbarriere, Beschichtung, Schwamm, antibakterieller Wundverband oder zur Verwendung bei Zellkultur, Sphäroidproduktion und Bildung embryonaler Körperchen.

## Revendications

1. Hydrogel obtenu par mise en réaction d'au moins une protéine de type collagène et d'au moins un agent de réticulation comprenant au moins deux groupes comprenant chacun un groupement polyalkylèneglycol et un groupe succinimidyle, dans lequel l'au moins une protéine de type collagène bactérienne est un polypeptide qui est au moins ≥ 60 %, identique à la séquence d'acides aminés de SEQ ID NO:1, SEQ ID NO:2, SEQ ID NO:3, SEQ ID NO:4, SEQ ID NO:5, SEQ ID NO:6, SEQ ID NO:7, SEQ ID NO:8 ou SEQ ID NO:9.

2. Hydrogel selon la revendication 1, dans lequel l'au moins une protéine de type collagène est une protéine de type collagène bactérienne.

3. Hydrogel selon la revendication 1 ou 2, dans lequel la protéine de type collagène bactérienne est une protéine de type collagène de *Streptococcus pyogenes,* de préférence Scl2.

4. Hydrogel selon l'une quelconque des revendications précédentes, dans lequel la protéine de type collagène bactérienne est produite par fermentation dans une cellule hôte, dans lequel la cellule hôte est une cellule de levure, de préférence *Pichia pastoris* ou une cellule bactérienne, de préférence *E. coli, Corynebacterium* ou *Brevibacterium.*

5. Hydrogel selon l'une quelconque des revendications précédentes, dans lequel l'agent de réticulation a l'une des formules (I) ou (II) suivantes : 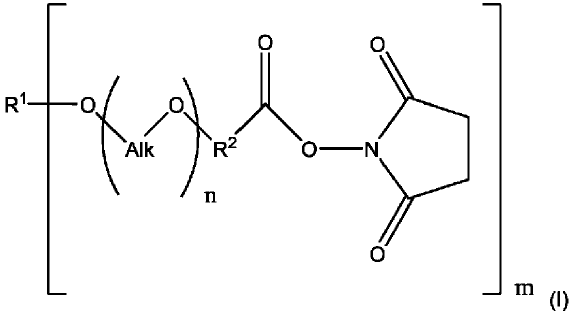 dans lequel
R¹ est un groupe alkyle linéaire ou ramifié ayant jusqu'à 12 atomes de carbone ;
Alk est -CH₂-, -CH₂-CH₂- ou -CH₂-CH₂-CH₂ ;
n est un entier de 1 à 1 350 ;
R² est -CH₂-, -C₂H₄-NH-(C=O)-C₃H₆-, -C₂H₄-O-, -C₂H₄-O-(C=O) - C₃H₆-, -C₂H₄-NH-(C=O)-C₂H₄- ou -C₂H₄-O-(C=O)-C₂H₄- ;
m est un entier de 2 à 8 ;
ou 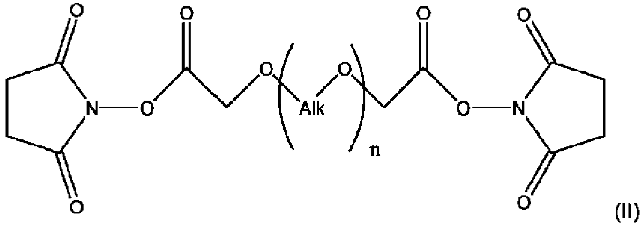
dans lequel
Alk est -CH₂-, -CH₂-CH₂- ou -CH₂-CH₂-CH₂- ; et
n est un entier de 1 à 1 350.

6. Hydrogel selon l'une quelconque des revendications précédentes, dans lequel la réaction a lieu dans un milieu aqueux ayant une valeur de pH de 6 à 8.

7. Hydrogel selon l'une quelconque des revendications précédentes, dans lequel la concentration en protéine de type collagène est de 1 à 100 mg/ml.

8. Hydrogel selon l'une quelconque des revendications précédentes, dans lequel le rapport molaire des groupes lysine dans la protéine de type collagène sur les groupes succinimidyle dans l'agent de réticulation est de 1:0,01 à 1:1,6.

9. Hydrogel selon l'une quelconque des revendications précédentes, dans lequel l'hydrogel est non adhésif.

10. Hydrogel selon l'une quelconque des revendications 1 à 9, pour utilisation comme stent, pour le scellement d'une plaie, l'apport d'un médicament, la favorisation de la cicatrisation, comme barrière anti-adhérence, revêtement, éponge, pansement antibactérien, ou pour utilisation dans une culture cellulaire, une production de sphéroïdes et une formation de corps embryoïdes.
